# EUROPEAN PATENT APPLICATION

(11) **EP 4 578 383 A1**
(43) Date of publication of application: **02.07.2025**
(21) Application number: 23383405.0
(22) Date of filing: 29.12.2023
(51) Int. Cl.: A61B 5/1477, H10K 10/40

(54) **DEVICE AND METHOD FOR BIOMARKERS PRESENCE AND/OR CONCENTRATION MONITORING AND USE THEREOF**

(71) Applicant: Fundación Tecnalia Research & Innovation, 20009 Donostia - San Sebastián (ES)
(72) Inventor: STRBAC, Matija, 20009 Donostia-San Sebastián (ES); BJELIC, Goran, 20009 Donostia-San Sebastián (ES); PEÑA, Marina, 20009 Donostia-San Sebastián (ES); BRIZ ICETA, Nerea, 20009 Donostia-San Sebastián (ES); OLALDE, Beatriz, 20009 Donostia-San Sebastián (ES); FERNÁNDEZ SAN ARGIMIRO, Francisco Javier, 20009 Donostia-San Sebastián (ES)
(74) Representative: Balder IP Law, S.L.

(57) **Abstract**

A device for monitoring the presence and/or concentration of a biomarker, comprising: a patch (10) intended to be fixed to a user's skin (30), which in turn, comprises: a semipermeable membrane (20), having a first face (21) and a second face (22), being the first face (21) intended to be disposed on a user's skin (30) during use of the device, wherein the semipermeable membrane (20) allows a controlled passage of a body fluid present on the user's skin, a first reverse iontophoretic electrode (41) and a second reverse iontophoretic electrode (42), in contact with the second face (22) of the semipermeable membrane (20), being the at least one first reverse iontophoretic electrode (41) and at least one second reverse iontophoretic electrode (42) configured to pass an electrical signal between them to create an electric field to drive a flow of biomarkers present in the body fluid through the semipermeable membrane (20), and a OECT (organic electrochemical transistor) sensor (50) biofunctionalized with a bioreceptor, disposed to receive the biomarkers flow and to generate a signal if the bioreceptor reacts with a biomarker. The device also comprises: at least one electrophysiological sensor (60), configured to obtain physiological information of the user, and processing means (71) configured to receive the signal from the OECT sensor (50) and the physiological information from the at least one electrophysiological sensor (60), and to determine the presence and/or quantity of a biomarker in the body fluid.

## Description

### TECHNICAL FIELD

The present invention relates to the detection, quantification and/or monitoring of biomarkers concentration, using biorecognition elements such as nanobodies or aptamers, and iontophoresis mechanisms. More specifically, the present invention relates to products and methods for such transcutaneous detection, quantification and/or monitoring.

### STATE OF THE ART

From wound healing to immuno-modulation, drug delivery and biomarkers monitoring, many therapeutic strategies rely on the application of electric fields and currents to and through the skin with an electronic device comprising skin-contacting electrodes.

One of the main applications of such devices is currently the non-invasive monitoring of biomarkers of interest, present in the body fluids of a user. In the development of new biomarkers monitoring devices based on transcutaneous techniques, the object is most often not only to detect said biomarkers, but also to quantify and continuously monitor their evolution in the user.

One of the possible techniques used for transcutaneous biomarkers monitoring is called reverse iontophoresis. Reverse iontophoresis is the application of a small electric current with modulations in pulse width and pauses to the skin. This process enhances molecular transport across the skin and sweat interface, facilitating the extraction of biomarkers.

Manipulation of either the total charge delivered and/or certain electrode formulation parameters allows control of electromigration and electroosmosis, the two principal mechanisms of transdermal reverse iontophoresis.

This non-invasive application represents an alternative to other more invasive, inefficient, and non-continuous monitoring methods, which can involve, for example, the use of needles to extract a blood sample.

Anyhow, this is a very interesting technique when it comes to its use in wearable devices. This is because it allows continuous monitoring of biomarkers and therefore, it can constantly detect and measure the biomarkers of interest of a user. Several wearable reverse iontophoresis-based devices have been proposed so far.

For example, document US11122982B2 discloses methods, systems, and devices for wearable, real-time multimodal sensing of electrochemical and electrophysiological and/or physical parameters of a user. In some aspects, the device comprises a reverse iontophoresis electrode that applies an electric field to drive an ion flow to monitor electrolytes and metabolites in the users' perspiration.

In turn, document US20210076988A1 discloses a non-invasive epidermal electrochemical sensor device that comprises a reverse iontophoretic electrode assembly that extracts interstitial fluids to the surface of the skin of the user and performs an electrochemical detection of a desired chemical analyte via electrochemical sensor electrodes.

However, to develop a wearable reverse iontophoresis-based sensor, there is a need to control when a suitable sensor must perform measurements in the fluid. Additionally, cross contamination during the collection of the fluid must also be avoided.

Therefore, there is a need to develop new wearable devices that overcome the drawbacks of the conventional ones.

### DESCRIPTION OF THE INVENTION

The drawbacks of conventional wearable devices are addressed by the present invention, which provides a device and method for monitoring biomarkers presence and/or concentration in a body fluid of a user.

In a first aspect of the invention, a device for monitoring of the presence and/or concentration of a biomarker is provided. The device comprises:
a patch intended to be fixed to a user's skin, which in turn, comprises:
a semipermeable membrane, having a first face and a second face, being the first face intended to be disposed on a user's skin during use of the device, wherein the semipermeable membrane allows a controlled passage of a body fluid present on the user's skin,
a first reverse iontophoretic electrode and a second reverse iontophoretic electrode, in contact with the second face of the semipermeable membrane, being the at least one first reverse iontophoretic electrode and at least one second reverse iontophoretic electrode configured to pass an electrical signal between them to create an electric field to drive a flow of biomarkers present in the body fluid through the semipermeable membrane,
a OECT (organic electrochemical transistor) sensor biofunctionalized with a bioreceptor, disposed to receive the biomarkers flow and to generate a signal if the bioreceptor reacts with a biomarker,
wherein the device additionally comprises:
at least one electrophysiological sensor configured to obtain physiological information of the user, and
processing means configured to receive the signal from the OECT sensor and the physiological information from the at least one electrophysiological sensor, and to determine the presence and/or quantity of a biomarker in the body fluid.

The reverse iontophoretic technique enables non-invasive sampling of analytes.

The at least one electrophysiological sensor can form part of the patch (for example, be integrated in the patch) or can be a separate element.

The device obtains the results of the presence and/or quantity of biomarkers in a periodical way, which can be, in an exemplary and non-limiting way, each 10 minutes, each 5 minutes, or each 1 minute. As the biomarkers presence and/or concentration in the body fluid is relatively stable in periods of time in the order of minutes (for example, in periods no longer than 10 minutes or in periods no longer than 5 minutes), obtaining the measurements with a maximum frequency of about 10 minutes, such as with a maximum frequency of about 5 minutes, or with a maximum frequency of 1 minute, enables to achieve a real time monitoring of the biomarkers.

A biomarker is a naturally occurring molecule, gene or characteristic by which a particular pathological or physiological processes or disease can be identified. In an exemplary and non-limiting way, some biomarkers that the device can detect and/or quantify, are: cortisol; ions; lactate; Cytokine proteins like TNFα, IL-1, IL-6, IL-8, IL-10, and other biomolecules.

Cortisol can be used for stress evaluation and other pathologies which can be a consequence of perdurable stress on time. Regarding cortisol, one relevant aspect in defining a person's state of health is the stress that the organism undergoes, mainly due to immunosuppressive and inflammatory effects (particularly in a sustained stress in time) stress responses have on individuals. It has been found that cortisol regulation of innate immunity can be both pro-inflammatory and anti-inflammatory. Associated with this, it is of vital importance to know and detect the immunological alterations produced by a physiological change in cortisol levels. Also, Cytokine proteins (TNFα, IL-1, IL-6, IL-8, IL-10...) are the main responsible for the defense mechanism of the immune system when a pathogen is detected in the organism, which triggers the secretion of a cascade of these proteins, considerably increasing their concentration in the blood. However, they also cause acute inflammatory processes in the case of inflammatory autoimmune diseases, where diseases of the digestive system (Crohn's disease and ulcerative colitis) and rheumatic diseases (rheumatoid arthritis and ankylosing spondylitis) are the most prevalent among others. Because of that, cytokine proteins are also considered biomarkers of interest.

A bioreceptor is a biomolecule which can identify a biological analyte. In an embodiment of the invention the bioreceptors are selected from the group formed by nanobodies and aptamers. These small bioreceptor molecules allow the detection on the OECTs without causing interferences due to the size of the biomolecules. The inventors have observed that OECTs detection methods are more reliable if the biorecognition elements are small than if some bigger biomolecules, such as antibodies, are used.

Biofunctionalizing refers to a surface modification by chemical methods to achieve some characteristics, for instance, that some molecules are bonded and others not.

A body fluid can be, among others, sweat and/or interstitial fluids. In an embodiment of the invention, biomarkers are measured from sweat (major component) and extracted interstitial fluid (minor component) of the fluid driven to the surface of the skin by natural and provoked (external stimuli) skin perspiration. Particularly, interstitial fluid is a fluid outside of blood vessels and outside of cells and some of this fluid will be present when iontophoretic stimuli is applied.

A patch is a piece of a non-rigid material or a combination of non-rigid materials, for example, but not limited to a piece of cloth or a plastic material, that can be worn on the skin of a user adapting to its shape. A patch can be formed by several layers of different materials.

The patch can be worn in any part of the body, for example, but not limited to, the arm and the back of a user.

The membrane being semipermeable means that it allows or restricts the body fluids diffusion. Body fluid permeability through the membrane can be controlled for example, by membrane solubility, swelling, pore diameter, fiber diameter or by external stimuli such as electrical field, temperature or pH.

In embodiments of the invention, the membrane is made of a water-soluble polymer, such as poly(vinyl alcohol) (PVA). In embodiments of the invention, the membrane is made of a water-degradable polymer, such as Poly-L-lactic acid (PLLA). For example, when the membrane is made of PVA or of PLLA, passive diffusion through the membrane occurs, enabling a certain control of the pass of the analyte through the membrane. This passive diffusion depends on the interaction of water with the membrane.

In embodiments of the invention, the membrane is made of poly (2-hydroxyethyl methacrylate) (pHEMA). The pHEMA membrane is preferably photopolymerized. The pHEMA membrane can be formulated with a cross-linker agent or without a cross-linker agent. When a pHEMA membrane with a cross-linker agent is formulated, certain passive diffusion through the membrane occurs. When a pHEMA membrane crosslinked without a cross-linker agent is used, controlled diffusion of the analyte is achieved through the application of electrical stimulus to move the analyte towards the OECT sensor. Therefore, this pHEMA membrane is preferably used. The pore size of the pHEMA membrane is in the order of angstrom (Å) (1 Å = 10⁻¹⁰ m), such as between 1 Å and 500 Å, for example between 1 Å and 200 Å, or between 1 Å and 100 Å, or between 1 Å and 50 Å.

Thus, the passage of the body fluid through the membrane is controlled in time by a controlled application of a iontophoretic current, which moves the body fluid (analyte) through the membrane, towards the OECT sensor. In other words, the selected membrane poses a barrier for passive diffusion of the biomarker and, by using iontophoretic current to control/trigger the diffusion of the analyte from the skin to the sensing element through the membrane, discrete timestamped values of the targeted biomarker can be measured.

In other words, a delayed start of the fluid collection and monitoring once the device has been placed on the skin of the user, is achieved thanks to the control on the iontophoretic current applied between anode and cathode electrodes and to the selected membrane.

On the other hand, OECT sensors offer several advantages with respect to other sensors. The inventors have observed that OECT sensors exhibit good mechanical stability and flexibility, making them suitable for applications where the biosensor may undergo bending or deformation. The inherent flexibility of these devices allows for integration into wearable either textile or plastic substrates, expanding their potential applications. Furthermore, they enable cost-effective and scalable manufacturing process, in turn enabled by the serigraphy technique which can be used for their production. This method allows for the mass production of OECT sensors, making them economically viable for large-scale biosensor applications. The serigraphic fabrication of OECTs also facilitates the integration of various electrodes, and/or biorecognition elements onto the transistor surface, such as the different electrophysiological sensors for pH, ionic strength, impedance and temperature, among others, enhancing the versatility of biosensor designs.

In an embodiment of the invention, the biomarker of interest is a cortisol. Cortisol is a biomarker that in healthy people varies during the day, being highest in the early morning and lowest before bedtime. The physiological relevant range of cortisol is 8.16 to 141.7 ng/ mL in perspired human sweat. In this case, a highly sensitive biosensor (with LODs (Limit of Detection) around 1-10ng/mL) is needed. In this regard, OECT sensors are established as the most sensitive transducers thanks to their operation principle.

In an embodiment of the invention, the processing means have embedded an artificial intelligence algorithm configured to determine the presence and/or concentration of the biomarker of interest based on the signal from the OECT sensor and the physiological information from the one or more electrophysiological sensors.

The artificial intelligence algorithm can periodically update its models based on new incoming data from the OECT sensor. This adaptive approach allows the device to improve its accuracy over time and adjust to individual variations. Over time, the artificial intelligence algorithm can tailor its models to individual users, taking into account each person's unique physiological responses and patterns which leads to more personalized and accurate assessments.

In an embodiment of the invention, the at least one electrophysiological sensor is selected from a group comprising temperature sensor, pH sensor, ionic strength sensor and impedance sensor, which generate a temperature measurement, a pH measurement, an ionic strength measurement and an impedance measurement respectively.

Temperature, pH, and ionic strength exert significant effects on the overall performance of immunoassays. Specifically, temperature affects reaction kinetics, molecular binding, and ion mobility in solution, impacting charge transfer rates and overall performance. Immunoassays typically have an optimal temperature range for maximum sensitivity, with deviations potentially altering reaction rates and performance. Regarding ionic strength, and referring to ion concentration in the solution, it affects the Debye length and the screening of charged species, influencing electrostatic interactions in the transistor channel. This parameter directly impacts sensitivity. The Debye length characterizes the distance over which the electric field of an ion or charged molecule is effectively screened by surrounding ions. Finally, regarding pH, it plays a crucial role in the ionization state of functional groups on the electrode surface, immobilized biomolecules, and the analyte charge. It can alter charge density and electrostatic interactions, thus influencing the binding of target biomolecules.

In an embodiment of the invention the processing means are further configured to control one or more of the following parameters of the electrical signal passed between the first and second reverse iontophoretic electrodes: current intensity, duration of current application and activation and deactivation of the reverse iontophoretic electrodes. This allows for more effective fluid collection, tailored to both the user's physiological state and biomarker monitoring at every instant. Controlling the one or more parameters enables not only the pass of the analyte through the membrane towards the OECT sensor, but also to control when the measurement should be made. When the activation of the electrical signal is made through the control of the mentioned one or more parameters, the analyte moves, passes through the membrane and reaches the sensor layer (OECT sensor), enabling the measurement.

The device comprises a current generator which is voltage-controlled. This enables to capture sensor measurements independently from the skin type, location of the patch on the skin and/or other possible interpatient variabilities. It also enables the application of iontophoretic stimulation patterns customized for different analytes. The iontophoretic stimulation patterns can be designed in such manner that skin irritation and skin damage is avoided.

In an embodiment of the invention the first and second reverse iontophoretic electrodes are concentric with respect to a central point. In this way, the electric current distribution is even from all sides, facilitating a homogeneous extraction of the body fluid.

In an embodiment of the invention, the OECT sensor is placed in the central point of the design formed by the first and second iontophoretic electrodes. In this way, as the electric current distribution is even from all sides around a focal point, which is the central point, the OECT sensor can achieve a better distributed and constant body fluid flow.

This disposition of the OECT sensor contributes to avoiding cross contamination of the analyte under measurement.

In an embodiment of the invention the pH sensor and/or the ionic strength sensor are placed in the central point. In this case, the pH sensor and/or the ionic strength sensor, like the OECT sensor, are in contact with the membrane in such a way that, when the membrane is moistened with the fluid, the corresponding sensor is allowed to measure the flux of the collected biofluid.

In an embodiment of the invention, the impedance sensor and/or the temperature sensor are placed outside the reverse iontophoretic electrodes. In this case, the impedance sensor and/or the temperature sensor can be disposed on the user skin, making direct contact with the skin or through a suitable, conventional conductive gel layer.

This allows for a more flexible device structure, where sensors that obtain measurements that vary rapidly along the user's skin are preferably placed on the central point, but where sensors that acquire measurements that do not vary significantly can be arranged on points on the user's skin that are most comfortable to the user.

In an embodiment of the invention, the device further comprises power supply means.

In this way, the device can be fully autonomous, facilitating its autonomy and wearability.

In an embodiment of the invention, the device further comprises a wireless communication module.

Thus, the device can send the results obtained to a mobile device or a computer, among others, for convenient checking.

In an embodiment of the invention, the device further comprises a display module.

In this way, the results can be shown on a display screen, both to the user and to a specialist.

In an embodiment of the invention, the device comprises two or more OECT sensors.

This embodiment allows multiple measurements to be obtained, e.g., at different points of the skin, which facilitates discarding atypical values, which could disrupt the results.

In an embodiment of the invention, the two or more OECT sensors are biofunctionalized with different bioreceptors.

This is a significant advantage, because in addition to the fact that these measurements can be taken at the same point or at different points on the skin, several biomarkers can be monitored simultaneously with a single device.

In a second aspect of the invention, a method for monitoring of biomarkers presence and/or concentration. The method comprising the steps of:
- providing:
   a first and second reverse iontophoretic electrodes, configured to pass an electrical signal between them to create an electric field to drive a flow of biomarkers present in a body fluid present on a user's skin, through a semipermeable membrane,
   a OECT sensor biofunctionalized with a bioreceptor, and
   processing means,
- receiving the biomarkers flow in the OECT sensor,
- generating, in the OECT sensor, a signal when the bioreceptor reacts with a biomarker, and
- feeding the signal to the processing means and obtaining data of a presence and/or concentration of a biomarker in the body fluid.

In an embodiment of the invention, the method additionally comprises:
- providing a pre-trained machine learning module in the processing means,
- providing at least one electrophysiological sensor selected from: a temperature sensor, a pH sensor, an ionic strength sensor and an impedance sensor, configured to generate a temperature measurement, a pH measurement, an ionic strength measurement and an impedance measurement respectively,
- feeding the signal from the OECT sensor and the measurements of the at least one electrophysiological sensor to the pre-trained machine learning module,
- obtaining, the pre-trained machine learning module, data of the presence and/or concentration of the biomarker in the body fluid.

In an embodiment of the invention, the method further comprises obtaining control parameters of a reverse iontophoretic current for the reverse iontophoretic electrodes.

The same advantages described for the first aspect of the invention can be applied to this second aspect of the invention.

A third aspect of the invention is the use of the reverse iontophoretic device of the first aspect of the invention in the monitoring of at least one biomarker selected from: cortisol, ions, lactate and Cytokine proteins like TNFα, IL-1, IL-6, IL-8, IL-10, among others.

Additional advantages and features of the invention will become apparent from the detail description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 illustrates a cross-sectional view of the device for biomarkers concentration monitoring, of the present invention.
Figure 2 shows an upper view of the device for biomarkers concentration monitoring.
Figure 3 illustrates a schematic representation of the electronics unit of the device of the invention.
Figure 4 shows a schematic representation of the OECT sensor.
Figure 5 illustrates a flow diagram of the method for monitoring of biomarkers concentration.

### DESCRIPTION OF A WAY OF CARRYING OUT THE INVENTION

The following description is not to be taken in a limiting sense but is given solely for the purpose of describing the broad principles of the invention. Next embodiments of the invention will be described by way of example, with reference to the above-mentioned drawings, showing apparatuses and results according to the invention.

Next, different embodiments of devices for biomarker's concentration monitoring, according to the invention, are disclosed.

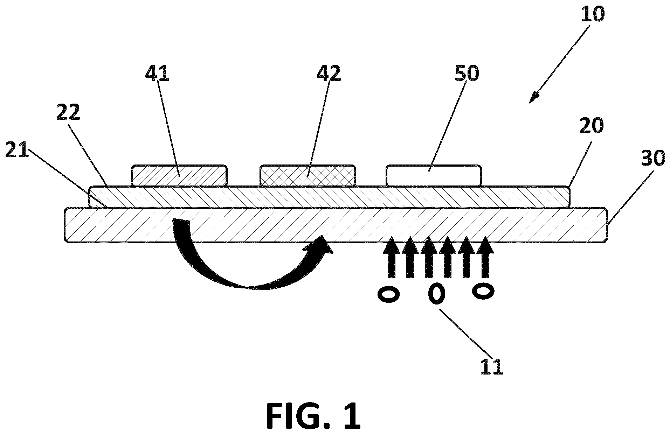

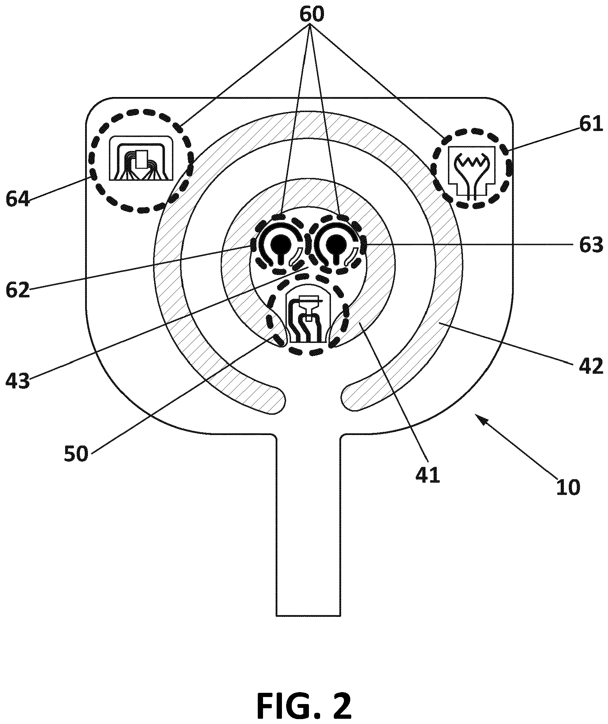

Figures 1 and 2 show a device for monitoring of biomarkers presence and/or concentration. In the context of this invention, monitoring includes, among others, detection and/or quantification of biomarkers 11 during a certain period. In this case, the device is in the form of a patch 10 because it is intended to be placed on the skin 30 of a user. The patch 10 is non-intrusive, as it facilitates the monitoring of one or more biomarkers 11 of interest in a user without additional tools, such as needles. The device being in the form of a patch 10 allows the user to wear it comfortably and during prolonged periods.

As shown in figure 1, the patch 10 comprises a semipermeable membrane 20, usually essentially flat, with a first face 21 and a second face 22. The membrane 20 is intended to be disposed on the user's skin 30. The membrane 20 being semipermeable means that it allows the passage of a certain amount of a fluid (a controlled passage of the analyte) with the aim of achieving prolonged monitoring and avoiding saturation due to an excess of analyte. Said fluid is a body fluid, such as, but not limited to, sweat or interstitial fluids. The membrane 20 is usually made of a flexible material, to facilitate its placement and adaptation to the skin 30 of the user.

As shown in figure 1, the patch 10 also comprises a fist and a second reverse iontophoretic electrodes 41, 42, being one an anode and the other one a cathode, placed on the second face 22 of the membrane 20, which is the one which, in use of the patch, is opposed to the skin 30 of the user. The iontophoretic electrodes 41, 42 are usually made of a flexible substrate, so they can also adapt to the user's skin 30. As the arrow in figure 1 shows, an electrical signal passes from one electrode 41 to the other one 42 creating an electric field to drive a flow of biomarkers, such as ions and/or biomolecules, in the body fluid.

Specifically, the passage of a low level of current through the skin 30 promotes the transport of both charged and neutral molecules. In the context of the present invention, the expression "low level" applied to the current refers to currents having a value comprised in the range of 500 - 2000 µA (1µA = 10⁻⁶ A). In terms of current density, "low level" refers to a current density < 0.5 mA/cm2. Two mechanisms are mainly involved: in the first one, direct interaction between charged species and applied electrical field generates the electromigration of them; the second one is related to the electroosmosis due to the induced solvent flow from anode to cathode. The movement of biofluid's components enables or generates the extraction of the flow of biomarkers from the body fluid.

The semipermeable membrane 20 enables a controlled passage of the body fluid (also referred to as analyte) from the skin to the side of the membrane 20 opposite the skin. Controlled passage means that, depending on the characteristics of the membrane 20 (for instance, membrane solubility, swelling, pore diameter, fiber diameter or by external stimuli such as electrical field, temperature, pH, etc.), it may take a certain time during which an electric current is applied until the body fluid begins to pass through the membrane 20, or in other cases, the membrane 20 may allow a limited flow of body fluid to pass through.

As shown in figure 1, the patch 10 also comprises an organic electrochemical transistor (OECT) sensor 50, which is associated with the reverse iontophoretic electrodes 41, 42, receiving the analyte flow (the analyte comprising biomarkers) once it has been extracted. The OECT sensor 50 is functionalized with a bioreceptor. In this way, then the OECT sensor 50 receives the analyte flow, the bioreceptor interacts with a biomarker of interest present in the analyte, generating a signal. The molecules or ions present in the body fluids (analyte) are moved through the membrane due to iontophoretic current and reach the biofunctionalized OECT provided with bioreceptor molecules. Once the biomarker gets the sensing layer on the surface of the OECT, specific bio-detection is done by electrochemical measurements. The bioreceptor molecules recognize the biomarker contained in the analyte. This biorecognition is transduced in a change in current.

The OECT sensor 50 is functionalized with a predefined bioreceptor, which creates a specific recognition interface for the target biomarker. When the sample, potentially containing the biomarker, is extracted, any binding interactions between the biomarker and the biorecognition element leads to a change in the electrical conductivity of the OECT sensor 50, which generates a signal.

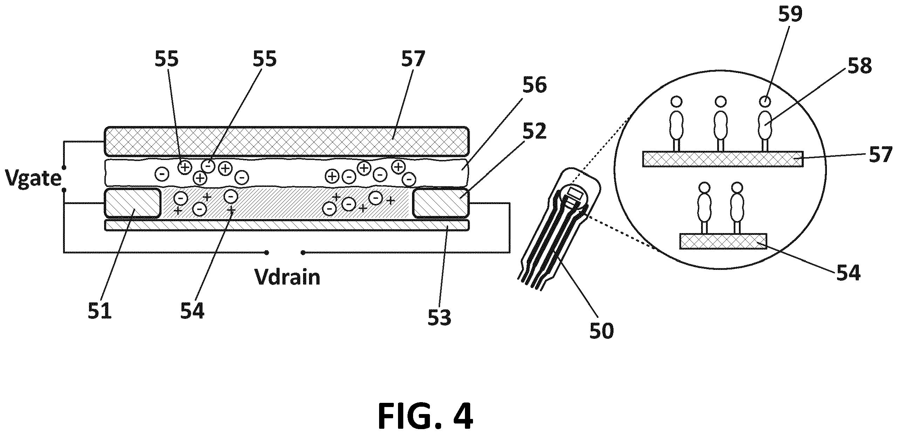

Particularly, in an exemplary and non-limiting way illustrated in figure 4, the OECT sensor 50 comprises a source electrode 51 and a drain electrode 52, which are connected to an organic semiconductor film 53, defining a channel 54 through which holes or electrons 55 flow from the source electrode 51 to the drain electrode 53. The film 53 is in contact with an electrolyte 56 contacting the circuit with a third electrode, a gate electrode 57. The OECT sensor 50 is functionalized with a predefined bioreceptor, which means that the bioreceptor (the biorecognition element) is immobilized in the channel/gate electrode. This immobilization creates a specific recognition interface for the target biomarker.

OECTs sensors 50 are based on the injection or extraction of biomarkers (such as ions or biomolecules) from the electrolyte 56 into the organic semiconductor film 53, altering the conductivity due to the change in the doping state. Small voltage signals applied to the gate electrode 57 are transduced into large changes in the drain 52 current. In the context of the present invention, the expression "small" applied to the voltage refers to voltage levels lower than ±3V, such lower than ±2V, for example voltage levels of about lower than ±1V. The transduction process is described by transfer curve which shows the dependence of the drain 52 current on the gate 57 voltage. The efficiency of transduction is called transconductance (gm) which is calculated by the first derivative of the transfer curve.

In figure 4, the OECT sensor 50 can be biofunctionalized with alfa-cortisol nanobodies 58, which recognizes specifically cortisol 59 when it is present in the ions flow, modifying the electrical conductivity of the OECT which then generates source 51-drain 52 current change. The normalized module of the source 51-drain 52 current depends on the amount of cortisol 59 detected.

In view of the above, the OECT sensor 50 is based on the principle of measuring the changes in electrical properties of a conductive material due to the absorption of an analyte on the surface functionalized with a biorecognition element.

In the case of the present invention, in use of the device, the bioreceptors are placed in the gate electrode 57, which is exposed to the sample, which contain the ions and biomarkers flow from the body fluid in this case. When the bioreceptor react with the biomarker 11, that is, a biorecognition reaction occurs, the gate 57 voltage changes depending on the biorecognition reaction. As a result, the source 51-drain 52 current flowing also changes, generating the aforementioned signal. More specifically, the biorecognition reaction is detected as a change in the source 51-drain 52 current at a fixed gate 57 voltage. Specific voltage level is first set to the electrode gate 57 which is placing the transistor in the working zone (for best sensitivity and linearity of the output). Physically, the change in the current in the channel results of ionic transfer (in case of semipermeable membrane 20, i.e. ion selective membranes) or due to capacitive effects and voltage gradient introduce by deposition of the bioreceptors.

As presented in figure 2, the OECT sensor 50 can be embedded in the reverse iontophoretic electrodes 41, 42. In this way, the body fluid extracted by the reverse iontophoretic electrodes 41, 42 passes directly to the OECT sensor 50, avoiding cross-contamination of the sample. As figure 2, illustrates, the OECT sensor 50 can be embedded in one of the reverse iontophoresis electrodes 41, 42, particularly it can be embedded in the anode. The operation is as follows: An electric current (preferably a pulsed electric current) flowing from one iontophoretic electrode to the other one, facilitates the transportation of biomarkers across the skin. During this process, the electric field generated aids in the conveyance of particular biomarkers, such as ions or biomolecules, through the skin and into a collection reservoir, in this case by passing through the membrane. The membrane can be engineered to allow the passage of specific biofluid and biomarkers, adjusting factors like its swelling capacity, pore size, fiber diameter, or in response to external stimuli. The biofluid then reaches the OECT sensor.

As previously explained, the OECT sensor 50 comprises a gate electrode 57, a source electrode 51 and a drain electrode 52. Several selections of gate electrode 57, source electrode 51 and drain electrode 52 materials have been tested. The present is a non-exhaustive list of the materials, in which the first one is the one corresponding to the gate 57, the second one to the source 51 and the third one to the drain 52: i) Ag, Ag, Ag; ii) Ag/AgCI, Ag, Ag; iii) Ag/AgCI, C, C; iv) C, Ag, Ag; v) C, C, C; vi) C, Ag/AgCI, Ag; vii) C, Ag/AgCI, C.

The patch 10 can be placed on the skin of an animal, such as a human being. The patch 10 can be placed in a multitude of areas of the user's body, for example, it may be placed on a skin 30 portion of an arm, leg, chest or spinal area of the body of a user. The shape of the patch 10 may be adapted to the shape of the specific body part. The patch 10 may be placed on the user's skin 30 such that at least some of the reverse iontophoresis electrodes 41, 42 make electrical contact with the skin 30. The reverse iontophoresis electrodes 41, 42 will then be in electrical contact, for example, with muscle tissue below the skin 30, being able to receive or transmit an electrical signal to the region of the muscle below the area of the skin 30 occupied by the reverse iontophoresis electrodes 41, 42. Thus, the electrodes 41,42 can provide through the skin 30 a current, for example to the muscle tissue, in order to extract the ions flow from the body fluid.

As represented in figure 1, the patch 10 of the invention constitutes a non-invasive solution to biomarkers 11 monitoring, as it uses reverse iontophoresis mechanisms to transport biomarkers 11 through the skin from body fluids. As previously explained, this technique uses electrical current to force the extraction of body fluids from the skin, without the need of needles, of any other kind of tools.

The reverse iontophoresis electrodes 41, 42 may be distributed in an arbitrary configuration. Alternatively, as shown in figure 2, the reverse iontophoresis electrodes 41, 42 may be concentric with respect to a central point 43. As it can be seen in figure 2, the reverse iontophoresis electrodes 41, 42 may have a substantial circular shape. In this case, the OECT sensor 50 may be placed in the central point 43 defined by the electrodes 41, 42. The body fluid can be, for example and in a non-limiting way, sweat, which is a rich source of relevant biomarkers.

As presented in figure 2, the patch 10 can comprise one or more electrophysiological sensors 60. Electrophysiological sensors 60 are represented in figure 2. They obtain data which can complement the measurements obtained with the OECT sensor 50, to provide better and more personalized results for each user. In particular, the electrophysiological sensors 60 can be, among others, a temperature sensor 61 that monitors the temperature of the user, a pH sensor 62 which determines the pH on the user's skin 30, an ionic strength sensor 63 which measures the strength of the electric field in the body fluid, and an impedance sensor 64, which obtains the impedance of the body fluid. All these measurements vary depending on the user and together with the OECT sensor's signal can be used to determine the user's state, particularly its physiological state like hydration, that can affect the results. Quantity of sweat, body temperature or strength of induced ionic current can all influence the estimated biomarker concentrations.

As shown in figure 2, when the reverse iontophoresis electrodes 41, 42 are concentric, the pH sensor 62 and /or the ionic strength sensor 63 can be placed on the central point 43 defined by the concentric electrodes 41, 42. This is because the pH of the skin 30 varies significantly between different areas and; the inventors have observed that, if accurate pH measurements which represents the pH of the skin 30 over which the patch 10 is placed, is desired, the pH sensor 62 must preferably be very close to the reverse iontophoretic electrodes 41, 42. The same applies to the ionic strength sensor 63 which, to obtain a representative measurement of the body fluid extracted, must preferably be placed close to the reverse iontophoretic electrodes 41, 42.

Regarding other electrophysiological sensors 60, as indicated in figure 2, such as impedance sensor 64 and/or temperature sensor 61, it/they can be placed inside or outside the iontophoresis electrodes 41, 42. In figure 2 they are placed outside the iontophoresis electrodes 41, 42. In fact, they can be placed far from the patch 10, in the most convenient place for the comfort of the user. The reason behind this placement is that both the impedance and the temperature do not vary significantly from one place to another on the body of the user, and therefore, an accurate measurement can usually be obtained in a multitude of places. Temperature impacts the kinetics of reactions (reaction/recognition rate) and the affinity between nanobodies and antigens. The strength of the bond between the biomolecules engaged in the assay can vary with temperature, influencing the specificity and sensitivity of the immunoassay. For instance, elevated temperatures can result in increased nonspecific binding and reduced specific binding, causing a shift in the entire calibration curve and altering sensitivity. Besides, intermolecular interactions can vary with temperature. Non-limiting examples of such intermolecular interactions include: inter molecular hydrogen bonds (not only bonding ionic covalent or coordination such as in complexes), different diffusion and convection, mobility of molecule, different adsorption kinetics, different chemical activity/affinity hence differences in reaction kinetics.

As represented in figure 2, one or more of the OECT sensor 50, the reverse iontophoretic electrodes 41, 42 and the one or more electrophysiological sensors 60 can be printed on a support layer and are in this sense in the same level. The support layer can be the membrane 20 or, in other cases, and additional support layer which is flexible and adapts to the user's skin 30. The different elements being printable is a great advantage, as the industrialization of the product is easier and cheaper.

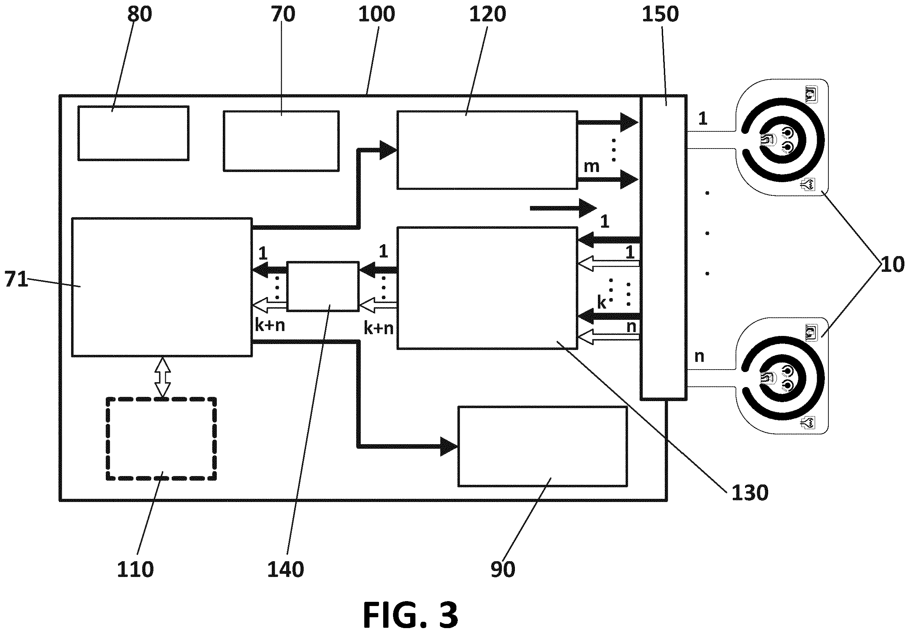

Figure 3 present the schematic representation of an electronics unit 100 of the device of the invention. Firstly, it comprises an interface (i.e. connector 150) to the patch (in particular, to the reverse iontophoretic electrodes 41, 42 and OECT sensor 50). The electronics unit 100 also comprises processing means 71, such as a microprocessor, which can be a standard component, and that receives the signal from the OECT sensor 50 via the connector 150. However, before arriving at the processing means 71, the signal is fed to a biosensing preprocessing module 130 and DAQ (data acquisition) module 140. The preprocessing module 130 conditions and filters the signals from the OECT sensor/s 50 and the DAQ module 140 samples and provides digital representation of the OECT sensor/s 50 status to the processing means 71, which oversees sensory data fusion.

Connected to the processing means 71, the electronics unit 100 also comprises an artificial intelligence module 110, with an integrated software whose function will be explained in more detail later, but which can calculate a biomarker's 11 concentration in real time, based on the signal from the OECT sensor 50 and the measurements from the electrophysiological sensor(s) 60.

To communicate the results to the user or to a specialist, the electronics unit 100 can also comprise a display module 70, a wireless communication module 90 or both. The display module 70 shows the data obtained in the patch 10, that is, the monitorization of the biomarker/s 11 of interest. The wireless communication module 90 sends to an external device, for example a computer or a mobile phone, said data. As shown in figure 3, the electronics unit 100 also comprise a power supply 80 which provides energy to the different elements, and which can be a standard component. Moreover, the electronics unit 100 also comprises a reverse iontophoretic current control module 120, connected between the processing means 71 and the reverse iontophoretic electrodes 41, 42. The reverse iontophoretic current control module 120 will be explained below in more detail. It allows the adaptation of the current applied to the user's skin by the reverse iontophoretic electrodes 41, 42 depending on the results obtained in the processing means 71, this is, like a feedback loop.

As it can be seen in the right side of figure 3, the device of the invention can comprise several patches 10, in this case from 1 to n all associated to the same electronics unit 100. This allows biomarker's 11 monitoring at different times. Alternatively, the patch 10 can comprise a single pair of reverse iontophoretic electrodes 41, 42 with several OECT sensors 50. In this case, the OECT sensor 50 can be biofunctionalized with the same bioreceptor or alternatively, with different bioreceptors. This allows the device to monitor several biomarkers 11. Additionally, the device can comprise several patches 10, where each patch 10 comprises more than one OECT sensors 50 biofunctionalized with different bioreceptors.

As previously indicated, the processing means 71 are connected to the OECT sensor 50, to receive the signal that it generates when the bioreceptor react with the biomarker 11 of interest of the body fluid. Having said signal, the processing means 71 determine the presence and quantity of the biomarker 11 of interest present in the body fluid.

The processing means 71 can monitor the presence and quantity of the biomarkers 11 once, to obtain a single measurement, routinely, with a given sampling period, or continuously, in such a way that the biomarker's presence and concentration is known during the whole time that the user is wearing the patch 10 and it is activated. This is both thanks to the processing means 71 that oversee the control of the patch 10, and thanks to the semipermeable membrane 20 which, as previously explained, facilitates a controlled flow of the body fluids.

When the patch 10 also comprises the one or more electrophysiological sensors 60, the measurements that they obtained are also send to the processing means 71, which combine them with the signal from the OECT sensor 50 to determine the presence and/or quantity of a biomarker present in the body fluid in a more personalized way.

The information provided by the electrophysiological sensors 60 relates to the physiological state of the user and the reverse iontophoretic extraction of the body fluid, which can influence the estimates of biomarker 11 concentration.

The artificial intelligence module 110 is integrated in the processing means 79 and, as previously indicated, it determines the presence of a biomarker and preferably calculates the biomarker's concentration in real time, based on the signal from the OECT sensor 50 and the measurements from the one or more coupling sensors 60. The artificial intelligence module 110 also can also provide a dynamic improvement of the obtained biomarker's concentration values via adapted and personalized assessment.

Specifically, the artificial intelligence module (also referred to as machine learning module) 110 implements sensory data fusion.

Parameters from the one or more coupling sensors 60, (the one or more coupling sensors 60 measure some parameters that will influence estimated concentration), are typically not linear. This causes a complex problem which is hard to solve directly/mathematically. The machine learning module 110 can be applied. The artificial intelligence module 110 applies regression models to estimate biomarker's concentrations based on the OECT sensor 50 and coupling sensor's 60 data. The artificial intelligence module 110 predicts a continuous output (the concentration of the biomarker/s) based on input features (OECT and coupling sensor data). The artificial intelligence 100 module is first trained on a dataset where the true biomarker's concentrations are known, learning the relationship between sensor data and biomarker levels and setting the parameters of the internal regression model. Furthermore, the artificial intelligence module 110 can continuously update its models based on new incoming data. This adaptive approach improves its accuracy over time and adjusts to individual variations. Over time, the artificial intelligence module 110 can tailor its models to individual users, taking into account each person's unique physiological responses and patterns which leads to more personalized and accurate assessments.

Finally, the reverse iontophoretic current control module 120 is connected to the processing means 71, and more particularly, to the artificial intelligence module 110, whose output can further include parameters to control the reverse iontophoretic current applied by the reverse iontophoretic electrodes 41, 42, adapting extraction of the body fluid to the physiological condition of the user estimated based on the data from the electrophysiological sensor 60, to further improve the accuracy and robustness of the concentration results. Depending on the results obtained in the processing means 71 they can send an order to the reverse iontophoretic current control module 120 to, for example, increase or decrease the current applied by the reverse iontophoretic electrodes 41,42 to the user's skin 30.

Specifically, they can control the current intensity, the duration of the current application and/or the activation or deactivation of the reverse iontophoretic electrodes 41, 42, between others.

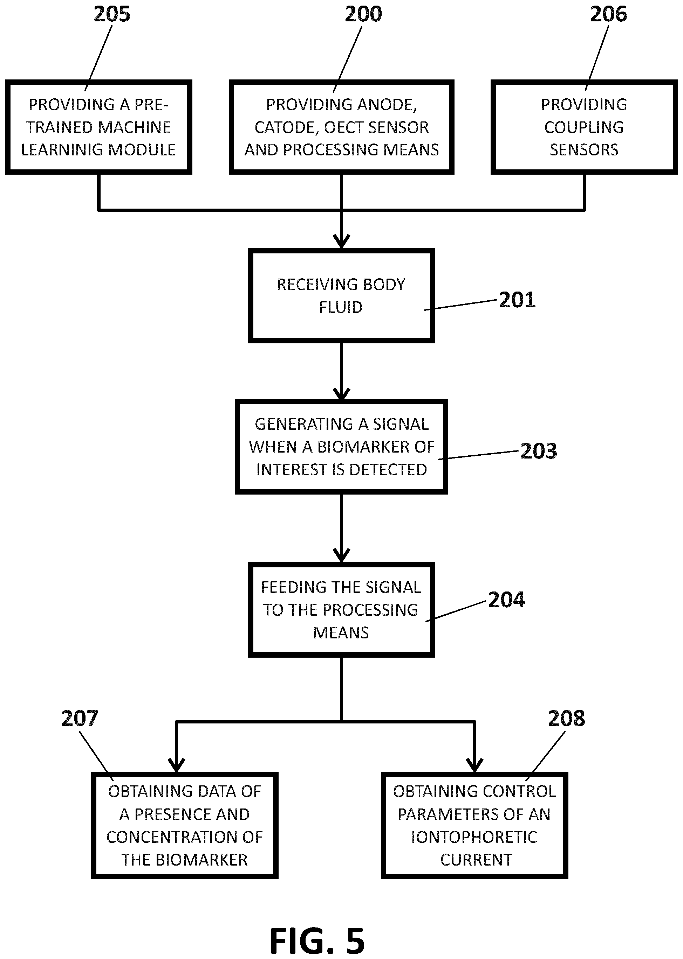

Finally, figure 5 illustrates a method for monitoring of biomarkers presence and/or concentration using a device comprising a reverse iontophoretic patch 10. As shown in the figure, in a first step 200, it is provided: a first and a second reverse iontophoretic electrodes 41, 42 (anode and cathode electrodes), configured to pass an electrical signal between them to create an electric field to drive ions flow in a body fluid present on a user's skin 30; an OECT sensor 50 biofunctionalized with a bioreceptor; and processing means 71. The method can also comprise providing a pre-trained 205 machine learning module 110 in the processing means 71, in the form, for example, of a machine learning algorithm. The method can also comprise a previous step of training the artificial intelligence algorithm.

To further complete the signals that are provided to the processing means, as shown in figure 5, the method can also comprise providing 206 one or more electrophysiological sensors. Examples of the one or more electrophysiological sensors 60 are: a temperature sensor 61, a pH sensor 62, an ionic strength sensor 63 and an impedance sensor 64, which generate a temperature measurement, a pH measurement, an ionic strength measurement and an impedance measurement respectively,
Following the flow diagram, the next step is receiving 201 a body fluid or analyte (which may comprise a biomarker, such as ions or biomolecules comprised in the body fluid) in the OECT sensor 50 and generating 203, in the OECT sensor 50, a signal when the bioreceptor reacts with a biomarker of interest present in the analyte.

Finally, the signal from the OECT sensor 50 and the measurements from the one or more electrophysiological sensors 60 are fed 204 to the processing means which obtain, using the artificial intelligence algorithm, data 207 of a presence and/or concentration of a biomarker in the body fluid.

The method can further comprise obtaining 208 control parameters of a reverse iontophoretic current for the reverse iontophoretic electrodes 41, 42, which are then applied to them.

In this text, the term "comprises" and its derivations (such as "comprising", etc.) should not be understood in an excluding sense, that is, these terms should not be interpreted as excluding the possibility that what is described and defined may include further elements, steps, etc.

In the context of the present disclosure, the term "approximately" and terms of its family (such as "approximate", etc.) should be understood as indicating values very near to those which accompany the aforementioned term. That is to say, a deviation within reasonable limits from an exact value should be accepted, because a skilled person in the art will understand that such a deviation from the values indicated is inevitable due to measurement inaccuracies, etc. The same applies to the terms "about", "around" and "substantially".

The invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.
Patch 10
Membrane 20
First face 21
Second face 22
Skin 30
First reverse iontophoretic electrode 41
Second reverse iontophoretic electrode 42
Central point 43
OECT sensor 50
Electrophysiological sensors 60
Temperature sensor 61
pH sensor 62
Ionic strength sensor 63
Impedance sensor 64
Display module 700
Processing means 71
Supply means 80
Communication module 90
electronics unit 100
Machine learning module 110
Providing step 200
Receiving step 201
Generating step 203
Feeding step 204
Providing a pre-trained machine learning module step 205
Providing at least a coupling sensor step 206
Obtaining data step 207
Obtaining control parameters 208

## Claims

1. A device for monitoring the presence and/or concentration of a biomarker, comprising:
a patch (10) intended to be fixed to a user's skin (30), which in turn, comprises:
a semipermeable membrane (20), having a first face (21) and a second face (22), being the first face (21) intended to be disposed on a user's skin (30) during use of the device, wherein the semipermeable membrane (20) allows a controlled passage of a body fluid present on the user's skin,
a first reverse iontophoretic electrode (41) and a second reverse iontophoretic electrode (42), in contact with the second face (22) of the semipermeable membrane (20), being the at least one first reverse iontophoretic electrode (41) and at least one second reverse iontophoretic electrode (42) configured to pass an electrical signal between them to create an electric field to drive a flow of biomarkers present in the body fluid through the semipermeable membrane (20),
a OECT (organic electrochemical transistor) sensor (50) biofunctionalized with a bioreceptor, disposed to receive the biomarkers flow and to generate a signal if the bioreceptor reacts with a biomarker,
wherein the device additionally comprises:
at least one electrophysiological sensor (60), configured to obtain physiological information of the user, and
processing means (71) configured to receive the signal from the OECT sensor (50) and the physiological information from the at least one electrophysiological sensor (60), and to determine the presence and/or quantity of a biomarker in the body fluid.

2. The device of claim 1, wherein the processing means (71) have embedded an artificial intelligence algorithm configured to determine the presence and/or concentration of the biomarker of interest based on the signal from the OECT sensor (50) and the physiological information from the at least one electrophysiological sensor (60).

3. The device of claims 1 or 2, wherein the at least one electrophysiological sensor (60) is selected from: a temperature sensor (61), a pH sensor (62), an ionic strength sensor (63) and an impedance sensor (64), configured to generate a temperature measurement, a pH measurement, an ionic strength measurement and an impedance measurement respectively.

4. The device of any preceding claims, wherein the processing means (71) are further configured to control one or more of the following parameters of the electrical signal passed between the first and second reverse iontophoretic electrodes (41, 42): current intensity, duration of current application and activation and deactivation of the reverse iontophoretic electrodes (41, 42).

5. The device according to any of the preceding claims, wherein the first and second reverse iontophoretic electrodes (41, 42) are concentric with respect to a central point (43).

6. The device of claim 5, wherein the OECT sensor (50) is placed in the central point (43).

7. The device of claims 3 and 6, wherein:
when the at least one electrophysiological sensor (60) comprises a pH sensor (62), the pH sensor (62) is placed in the central point (43),
when the at least one electrophysiological sensor (60) comprises an ionic strength sensor (63), the ionic strength sensor (63) is placed in the central point (43),
when the at least one electrophysiological sensor (60) comprises an impedance sensor (64), the impedance sensor (64) is placed outside the reverse iontophoretic electrodes (41, 42), or
when the at least one electrophysiological sensor (60) comprises a temperature sensor (61), the temperature sensor (61) is placed outside the reverse iontophoretic electrodes (41, 42).

8. The device of any preceding claims, further comprising power supply means (80) and/or a wireless communication module (90) and/or a display module (100).

9. The device of any preceding claims, wherein the bioreceptor is selected from the following list: nanobodies and aptamers.

10. The device of any of the preceding claims, wherein it comprises two or more OECT sensors (50).

11. The device of claim 10, wherein the two or more OECT sensors (50) are biofunctionalized with different bioreceptors.

12. A method for monitoring of biomarkers presence and/or concentration, comprising:
- providing (200):
a first and second reverse iontophoretic electrodes (41, 42), configured to pass an electrical signal between them to create an electric field to drive a flow of biomarkers present in a body fluid present on a user's skin (30), through a semipermeable membrane (20),
a OECT sensor (50) biofunctionalized with a bioreceptor, and
processing means (71),
- receiving (201) the biomarkers flow in the OECT sensor (50),
- generating (203), in the OECT sensor (50), a signal when the bioreceptor reacts with a biomarker, and
- feeding (204) the signal to the processing means (71) and obtaining data (207) of a presence and/or concentration of a biomarker in the body fluid.

13. The method of claim 12, wherein it additionally comprises:
- providing a pre-trained (205) machine learning module (110) in the processing means (71),
- providing (206) at least one electrophysiological sensor (60) selected from: a temperature sensor (61), a pH sensor (62), an ionic strength sensor (63) and an impedance sensor (64), which generate a temperature measurement, a pH measurement, an ionic strength measurement and an impedance measurement respectively,
- feeding (204) the signal from the OECT sensor (50) and the measurements of the at least one electrophysiological sensor (60) to the pre-trained machine learning module (110),
- obtaining, the pre-trained machine learning module (110), data of the presence and/or concentration of the biomarker in the body fluid.

14. The method of claims 12 or 13, wherein it further comprises obtaining (208) control parameters of a reverse iontophoretic current for the reverse iontophoretic electrodes (41, 42).

15. Use of the reverse iontophoretic patch (10) of any one of claims 1 to 11 in the monitoring of at least one biomarker selected from: cortisol; ions; lactate and Cytokine proteins.
